# EUROPEAN PATENT APPLICATION

(11) **EP 0 705 821 A1**
(43) Date of publication of application: **10.04.1996**
(21) Application number: 95202620.1
(22) Date of filing: 29.09.1995
(51) Int. Cl.: C07D 213/89, C07D 217/02, C07D 241/52, C07D 215/60, C07D 213/68, C07D 213/61

(54) **Oxidation of nitrogen heterocycles and heterocyclic N-oxides produced thereby**

(30) Priority: 06.10.1994 FR 9412051
(71) Applicant: SOLVAY INTEROX (Société Anonyme), B-1050 Bruxelles (BE)
(72) Inventor: Battioni, Pierrette, F-91300 Massy (FR); Mansuy, Daniel, F-75015 Paris (FR); Thellend, Annie, F-75018 Paris (FR); Sanderson, William R., Penketh WA5 2PE (GB)
(74) Representative: Decamps, Alain René François

(57) **Abstract**

A process for the production of aromatic heterocyclic N-oxides by reaction between an aromatic compound comprising a heterocyclic nitrogen atom and hydrogen peroxide in the presence of a manganese porphyrin catalyst is provided. The manganese porphyrin catalyst is substituted by an aromatic group at each meso-position, the aromatic nuclei of the aromatic substituents being substituted by one or more electron-withdrawing groups and/or comprising an aromatic quaternary ammonium heteroatom in the aromatic ring. Preferably, the manganese porphyrin catalyst is manganese tetrakis (2,6-dichlorophenyl) porphyrin chloride, manganese tetrakis (2,6-dichlorophenyl-4-sulphonic acid) porphyrin chloride, manganese tetrakis (4-methylpyridinium) porphyrin chloride, or manganese tetrakis (pentafluorophenyl) porphyrin chloride.

## Description

This invention concerns a process for the oxidation of aromatic compounds comprising heterocyclic nitrogen atoms. More specifically, this invention concerns the catalysed oxidation by hydrogen peroxide of aromatic compounds comprising heterocyclic nitrogen atoms to produce heterocyclic N-oxides, and heterocyclic N-oxides produced thereby.

Heterocyclic N-oxides are widely employed as intermediates or finished products in the chemical industry, particularly in the agrochemical and pharmaceutical industries. Heterocyclic N-oxides can conveniently be produced by the oxidation of the corresponding heterocyclic aromatic compound. This oxidation can be achieved by the use of peroxygen compounds. Reagents that can be successfully employed for the production of heterocyclic N-oxides include peracetic acid and meta-chloroperbenzoic acid.

Although effective, these reagents are relatively expensive and can give rise to problems on account of the organic residue remaining after their use.

Additionally, the N-oxide product is obtained in the form of a salt which can make the work up of the reaction mixture difficult. To avoid such problems, it would be desirable to employ hydrogen peroxide as oxidant, which leaves only water as the residue after oxidation. However, hydrogen peroxide by itself is relatively unreactive towards aromatic heterocyclic nitrogen atoms. The reactivity of hydrogen peroxide towards aromatic heterocyclic nitrogen atoms can be increased by the use of transition metal catalysts such as molybdates or tungstates, for example, the processes described by Syper et al, Synthesis Communications, 1979, pp. 521 - 2. These processes often have the disadvantage that relatively forcing conditions are still required, for example, a high temperature and/or a long reaction time.

It is an object of the present invention to provide a further process for the production of heterocyclic N-oxides by the oxidation with hydrogen peroxide of aromatic compounds comprising heterocyclic nitrogen atoms.

According to the present invention, there is provided a process for the production of heterocyclic N-oxides by reaction between an aromatic compound comprising a heterocyclic nitrogen atom and hydrogen peroxide in the presence of a catalyst, characterised in that the catalyst comprises a manganese porphyrin bearing an aromatic substituent at each meso-position, the aromatic nucleus of the aromatic substituents being substituted by one or more electron-withdrawing groups and/or comprising an aromatic quaternary ammonium heteroatom in the aromatic nucleus.

Aromatic compounds comprising heterocyclic nitrogen atoms that can be oxidised by the process according to the present invention comprise one or more heterocyclic nitrogen atoms in the aromatic ring. The aromatic ring may be substituted by one or more substituents. The process according to the present invention is particularly suitable for the oxidation of aromatic compounds comprising heterocyclic nitrogen atoms which are not substantially deactivated to nitrogen oxidation. It will be recognised that such deactivation can occur by one or both of electronic effects, caused by the presence of electron withdrawing substituents, such as nitro groups, and steric effects hindering the accessibility of the heterocyclic nitrogen atom to oxidation, such as the presence of bulky substituents at one or both positions adjacent to the heterocyclic nitrogen atom. Aromatic compounds comprising heterocyclic nitrogen atoms which are not substantially deactivated to nitrogen oxidation include unsubstituted compounds and those substituted by one or more electron donating and neutral groups, such as hydroxy, alkoxy, alkyl, particularly short chain, for example C1 to C4 alkyl, and amino groups. It will be recognised that the aromatic ring comprising the heterocyclic nitrogen atom can be substituted by one or more other aromatic rings, which themselves may comprise heteroatoms. Examples of heterocyclic compounds that can be oxidised by the process according to the present invention include pyridine; alkylpyridines, for example, 2-methylpyridine, 4-methylpyridine, 2-ethyl-5-methylpyridine; hydroxyalkylpyridines, for example 4-hydroxymethylpyridine; and quinolines, for example quinoline and isoquinoline.

The catalyst employed in the process according to the present invention comprises a manganese porphyrin bearing aromatic substituents at each meso-position, the aromatic rings of the aromatic substituents being substituted by one or more electron-withdrawing groups and/or comprising an aromatic quaternary ammonium heteroatom in the aromatic ring. The substituents at the meso-positions can be different substituents, but in many embodiments, the substituents will be the same at each meso-position. It will be recognised that the porphyrin ring is itself potentially oxidised and degraded by oxidation. Without wishing to be bound by any theory, it is believed that, in many embodiments, the selection of catalysts of the instant invention increases the resistance of the porphyrin itself to oxidation without reducing its activity as a catalyst in the instant process.

The aromatic substituents at the meso-positions of the porphyrins can comprise one or more, commonly 6-membered, aromatic rings, but normally comprise only one such aromatic ring. Preferably, the aromatic ring is a benzene ring or a pyridinium ring. When the substituent's aromatic ring does not comprise a quaternary ammonium heteroatom, electron-withdrawing substituents on the ring comprise one or more of halo groups, such as fluoro, chloro and bromo groups, particularly chloro groups, nitro groups, sulphonic acid groups, alkylcarboxy groups and quaternary ammonium groups. The electron withdrawing substituents can be present at any position on the substituent's aromatic ring, but will most commonly be at one or more of the positions ortho or para to the bond to the meso position. When the aromatic ring comprises a quaternary ammonium heteroatom, the aromatic ring need not be substituted by an electron-withdrawing substituent. However, electron withdrawing substituents may be present if desired. The aromatic ring comprising a quaternary ammonium heteroatom may optionally be substituted by one or more neutral substituents such as alkyl, particularly short chain, for example C1 to C4 alkyl groups. The quaternary ammonium heteroatom can be present at any position in the aromatic ring, but will most commonly be at one of the positions ortho or para to the bond to the meso position. In addition to the substituents at the meso-positions, the manganese porphyrins can additionally be substituted at one or more of the beta-positions. The manganese porphyrins according to the present invention are commonly employed as salts including halide salts, carboxylates and methanesulphonates, with chloride salts being preferred. Examples of suitable catalysts include manganese tetrakis (2,6-dichlorophenyl) porphyrin chloride, manganese tetrakis (2,6-dichlorophenyl-4-sulphonic acid) porphyrin chloride, manganese tetrakis (4-methylpyridinium) porphyrin chloride and manganese tetrakis (pentafluorophenyl) porphyrin chloride.

The mole ratio of substrate aromatic compounds comprising a heterocyclic nitrogen atom : manganese porphyrin catalyst will be selected depending for example on the reactivity of the substrate and the activity of the catalyst, and is often selected to be in the range of from 10 : 1 to 1000 : 1. In certain embodiments of the present invention, good results have been achieved employing a mole ratio of substrate aromatic compound comprising a heterocyclic nitrogen atom : manganese porphyrin catalyst in the range of from 100 : 1 to 600 : 1.

In addition to the manganese porphyrin catalyst, in many embodiments of the present invention, it is desirable to employ one or more co-catalysts. Without wishing to be bound by any theory, it is believed that the oxidation process according to the present invention proceeds via the formation of a selectively-reactive manganese-oxo species by homolytic cleavage of a manganese-hydroperoxy species, the leaving group being an hydroxide group. It is therefore desirable to promote such homolytic fission in preference to heterolytic cleavage, as this latter produces a non-selective radical species which can oxidise and degrade the porphyrin and/or rapidly decompose the hydrogen peroxide. The function of the co-catalyst in the process according to the present invention is believed to be to promote the homolytic cleavage. This can be achieved by one or both of partially or fully ligating to the manganese porphyrin complex, thus effectively donating electrons to the metal, or by acid-base activity facilitating the leaving of the hydroxide group. Examples of suitable co-catalysts include ammonium carboxylates, particularly ammonium acetate, imidazole, aliphatic N-oxides such as triethylamine N-oxide, and carboxylic acids, such as benzoic and lauric acid.

The mole ratio of manganese porphyrin catalyst to co-catalyst will vary depending on the activities of the catalysts and the reactivity of the substrate, and is often selected to be in the range of from 1 : 1 to 1 : 100. In certain embodiments, good results have been achieved employing a mole ratio of manganese porphyrin catalyst to co-catalyst in the range of from 1 : 5 to 1 : 50.

The process according to the present invention can be carried out in either a one or two phase system. When a two phase system is employed, a phase transfer catalyst such as a quaternary ammonium halide can be employed. Preferably, the process according to the present invention is carried out in a one phase system. Although the aromatic compound comprising a heterocyclic nitrogen atom may be able to serve as its own solvent, it will often be desirable or necessary to employ one or more additional solvents. In many embodiments of the present invention, the aromatic compound comprising a heterocyclic nitrogen atom will be soluble in hydrophobic solvents but only partially soluble in water, whereas the hydrogen peroxide will be substantially insoluble in hydrophobic solvents. It is therefore desirable to employ a mixture of a hydrophobic solvent and a hydrophilic solvent at suitable ratios to produce a single phase system when mixed. The solvents chosen should preferably be substantially resistant to oxidation under the reaction conditions employed. Examples of suitable hydrophobic solvents include C1 to C4 chlorinated aliphatic compounds, particularly dichloromethane, chloroform and 1,2-dichloroethane, with dichloromethane being preferred. Examples of hydrophilic solvents include low molecular weight nitriles such as acetonitrile, oxidation resistant alcohols such as methanol and t-butanol, N-alkylated lactams such as N-methyl pyrrolidone, and N-alkylated cyclic ureas such as N,N'-dimethylimidizolidinone. The most preferred hydrophilic solvent is acetonitrile.

Without prejudice to the generality of the preceding paragraph, in certain embodiments of the present invention, when a water soluble manganese porphyrin catalyst is employed and the aromatic compound comprising a heterocyclic nitrogen atom is at least partially soluble in water, water can comprise at least part of the solvent. In such a case, the pH of the reaction mixture is preferably about neutral pH, such as from pH 6 to about pH 8.

When a combination of a hydrophobic solvent and a hydrophilic solvent is employed, the weight ratio of hydrophobic solvent to hydrophilic solvent will often be in the range of from about 10 : 1 to 1 : 10, although weight ratios outside this range can be employed if required to achieve the desired solubility. In many embodiments, the weight ratio of hydrophobic solvent to hydrophilic solvent is in the range of from about 3 : 1 to 1 : 3.

Hydrogen peroxide is conveniently employed in the process according to the present invention either in the form of an aqueous solution or as an aqueous solution diluted with a hydrophilic organic solvent. Suitable hydrophilic solvents are those discussed above as additional solvents. Preferably, when the aqueous solution of hydrogen peroxide diluted by a hydrophilic solvent and a hydrophilic solvent is employed as additional solvent, the same hydrophilic solvent is employed. The concentration of hydrogen peroxide in the aqueous solution when not diluted further with a hydrophilic organic solvent is commonly from about 20% to about 90% by weight, with concentrations in the range of from about 25% to about 75% by weight, particularly from about 28% to about 55% by weight being preferred. When the hydrogen peroxide is employed as an aqueous solution diluted with a hydrophilic organic solvent, the concentration of hydrogen peroxide based on the diluted solution is often in the range of from 1% to 15% by weight. In certain embodiments of the present invention, good results have been achieved with hydrogen peroxide concentrations in the range of from 1% to about 7% by weight. Preferably, the hydrogen peroxide is introduced into a reaction mixture which contains both the heterocyclic compound and the catalyst, and particularly preferably it is introduced gradually, for example over a period of from 15 minutes to 6 hours, such as in small increments or in a continuous feed.

The mole ratio of hydrogen peroxide to oxidisable heterocyclic nitrogen atom can vary over a wide range. Although the mole ratio of hydrogen peroxide to oxidisable heterocyclic nitrogen atom is often stoichiometric, i.e. 1 : 1, or greater, it will be recognised that substoichiometric mole ratios can be employed, for example when the substrate contains other functional groups which are sensitive to oxidation. In many embodiments, it is preferred to employ a greater than stoichiometric mole ratio to allow for decomposition of hydrogen peroxide that may occur. The mole ratio of hydrogen peroxide to oxidisable heterocyclic nitrogen atom is commonly in the range of from 1 : 1 to 10 : 1, and particularly from 2 : 1 to 6 : 1.

The process according to the present invention is often carried out at a temperature in the range of from about ambient temperature, e.g. 15 to 25°C, to about 75°C, and is commonly less than 60°C. The reaction between the hydrogen peroxide and the substrate is normally continued until the required extent of reaction is reached. The reaction time will depend on the reaction conditions, particularly the reaction temperature. In many embodiments, the reaction time will be in the range of from 2 hours to 24 hours.

On completion of the reaction, the manganese porphyrin catalyst may be separated from the reaction mixture by conventional techniques, such as evaporation of solvents and distillation of products. The catalyst may then be employed in further oxidations.

According to a preferred aspect of the present invention, there is provided a process for the production of heterocyclic N-oxides by reaction between an aromatic compound comprising a heterocyclic nitrogen atom and hydrogen peroxide in the presence of a catalyst, a co-catalyst, and an organic solvent, characterised in that the catalyst comprises a manganese porphyrin selected from the group consisting of manganese tetrakis (2,6-dichlorophenyl) porphyrin chloride, manganese tetrakis (2,6-dichlorophenyl-4-sulphonic acid) porphyrin chloride and manganese tetrakis (4-methylpyridinium) porphyrin chloride, the co-catalyst is ammonium acetate and that the solvent is a mixture of dichloromethane and acetonitrile.

Having described the invention in general terms, specific embodiments thereof are described in greater detail by way of example only.

### Example 1. Preparation of Pyridine N-oxide.

Manganese tetrakis (2,6-dichlorophenyl) porphyrin chloride (Mn(TDCPP)Cl) (0.05 mmol), ammonium acetate (0.5 mmol) and pyridine (12.5 mmol) were dissolved in 21 mls of a 1 : 1 v/v mixture of dichloromethane and acetonitrile. To this solution was added, with stirring at ambient temperature, 3% hydrogen peroxide solution produced by diluting 30% w/w aqueous hydrogen peroxide 10 times with acetonitrile. The hydrogen peroxide was added over 6 hours by peristaltic pump. On completion of the hydrogen peroxide addition, the reaction mixture was stirred overnight. The solvent was then evaporated under reduced pressure and the residue freeze dried. The residue was purified by chromatography using silica gel (70 - 230 mesh ASTM) commercially available from Merck under the tradename Kieselgel SI60 with chloroform/methanol (95/5) eluent. The catalyst was recovered substantially intact, and pyridine N-oxide obtained in 87% yield.

### Example 2. Preparation of isoquinoline N-oxide

The general procedure of Example 1 was followed, except that 2 µmol Mn(TDCPP)Cl, 22 µmol ammonium acetate, 3 mmol hydrogen peroxide, 1 mmol isoquinoline and 1.7 mls of 1:1 v/v mixture of dichloromethane and acetonitrile were employed. After freeze drying of the residue, the residue was dissolved in D₂O and analysed by proton NMR with methanol as internal standard. 81% of the isoquinoline was converted, with a yield of isoquinoline N-oxide of 72%.

### Comparisons 3 and 4 and Examples 5 to 23

In these Comparisons and Examples, the general procedure of Example 2 was followed, but the addition time of the hydrogen peroxide solution was 3 hours. The substrate, manganese porphyrin catalyst, and mole ratio of hydrogen peroxide to substrate and the % yield of N-oxide (based on the amount of starting material) obtained are given in Table 1 below. In Table 1, the presence or absence of ammonium acetate co-catalyst is indicated by "yes" or "no". n/d indicates that the yield of product, if any, was unquantifiably small.

**TABLE 1**

| Example/Comparison | Substrate | Mn Porphyrin Catalyst | Cocatalyst H₂O₂:substrate | Mole ratio | % Yield |
|---|---|---|---|---|---|
| 3 | pyridine | Mn(TPP)Cl | No | 3.0 | n/d |
| 4 | pyridine | Mn(TPP)Cl | Yes | 3.0 | n/d |
| 5 | pyridine | Mn(TDCPP)Cl | No | 3.0 | 84 |
| 6 | pyridine | Mn(TDCPP)Cl | Yes | 3.0 | 88 |
| 7 | pyridine | Mn(TFPP)Cl | Yes | 3.0 | 48 |
| 8 | isoquinoline | Mn(TDCPP)Cl | No | 3.0 | 89 |
| 9 | isoquinoline | Mn(TDCPP)Cl | Yes | 3.0 | 94 |
| 10 | isoquinoline | Mn(TFPP)Cl | Yes | 3.0 | 39 |
| 11 | quinoxaline | Mn(TDCPP)Cl | Yes | 3.0 | 27 |
| 12 | isoquinoline | Mn(TDCPP)Cl | Yes | 2.5 | 94 |
| 13 | quinoline | Mn(TDCPP)Cl | Yes | 4.0 | 86 |
| 14 | 2-picoline | Mn(TDCPP)Cl | Yes | 2.0 | 81 |
| 15 | 2-ethyl-5-methylpyridine | Mn(TDCPP)Cl | Yes | 3.0 | 87 |
| 16 | 4-hydroxymethylpyridine | Mn(TDCPP)Cl | Yes | 2.0 | 85 |
| 17 | quinoxaline | Mn(TDCPP)Cl | Yes | 5.0 | 33 |
| 18 | pyridine | Mn(TDCPP)Cl | Yes | 1.5 | 82 |
| 19 | isoquinoline | Mn(TDCPP)Cl | Yes | 2.0 | 87 |
| 20 | isoquinoline | Mn(TDCPP)Cl | Yes | 2.5 | 95 |
| 21 | 2-picoline | Mn(TDCPP)Cl | Yes | 3.0 | 83 |
| 22 | 4-picoline | Mn(TDCPP)Cl | Yes | 2.0 | 86 |
| 23 | 4-chloropyridine | Mn(TDCPP)Cl | Yes | 5.0 | 33 |

In Table 1 above, the details of the catalysts are as follows:
Mn(TPP)Cl represents manganese tetraphenylporphyrin chloride
Mn(TDCPP)Cl represents manganese tetrakis (2,6-dichlorophenyl) porphyrin chloride.
Mn(TFPP)Cl represents manganese tetrakis (pentafluorophenyl) porphyrin chloride.

The results in Table 1 demonstrate that the process according to the present invention can be successfully used to produce the N-oxides of a range of heterocyclic aromatic compounds comprising a heterocyclic nitrogen atom. The results of Comparisons 3 and 4 show that when a manganese porphyrin catalyst not according to the present invention is employed, even in the presence of a co-catalyst, the yield of N-oxide, if any, was too low to be quantified. In Examples 5 and 6, which correspond to Comparisons 3 and 4, the use of a manganese porphyrin catalyst according to the present invention, gave good yields of N-oxide, particularly when ammonium acetate was employed as co-catalyst.

### Examples 24 and 25. Use of water as solvent

In these Examples, pyridine and 4-picoline (250 µmol) were oxidised at pH 7 and 7.4 respectively, in the presence of manganese tetrakis (2,6-dichlorophenyl-4-sulphonic acid) porphyrin chloride (2 µmol), ammonium acetate (22 mmole) in water (2 mls) at room temperature by the addition, with stirring, of hydrogen peroxide (1000 µmol) as a 3% hydrogen peroxide solution produced by diluting 30% w/w aqueous hydrogen peroxide 10 times with acetonitrile. The hydrogen peroxide was added over 6 hours by peristaltic pump. On completion of the hydrogen peroxide addition, the reaction mixture was stirred overnight. The mixture was then extracted with deuterated chloroform (2 x 1 ml) and the yield of N-oxide calculated by H¹ NMR against 1,1,2,2-tetrachloroethane as internal standard. The yields of N-oxide achieved based on the amount of starting material were 21% and 28% respectively.

The results of these Examples demonstrate that water can be used as the solvent.

## Claims

1. A process for the production of heterocyclic N-oxides by reaction between an aromatic compound comprising a heterocyclic nitrogen atom and hydrogen peroxide in the presence of a catalyst, characterised in that the catalyst comprises a manganese porphyrin bearing an aromatic substituent at each meso-position, the aromatic nucleus of the aromatic substituents being substituted by one or more electron-withdrawing groups and/or comprising an aromatic quaternary ammonium heteroatom in the aromatic nucleus.

2. A process according to claim 1, characterised in that the electron withdrawing groups are selected from halo groups, nitro groups, sulphonic acid groups, alkylcarboxy groups and quaternary ammonium groups.

3. A process according to either preceding claim, characterised in that the manganese porphyrin catalyst is selected from the group consisting of salts of manganese tetrakis (2,6-dichlorophenyl) porphyrin, manganese tetrakis (2,6-dichlorophenyl-4-sulphonic acid) porphyrin and manganese tetrakis (4-methylpyridinium) porphyrin.

4. A process according to claim 3, characterised in that the manganese porphyrin catalyst is selected from the group consisting of manganese tetrakis (2,6-dichlorophenyl) porphyrin chloride, manganese tetrakis (2,6-dichlorophenyl-4-sulphonic acid) porphyrin chloride and manganese tetrakis (4-methylpyridinium) porphyrin chloride.

5. A process according to any preceding claim, characterised in that the process employs ammonium acetate as co-catalyst.

6. A process according to claim 5, characterised in that the mole ratio of manganese porphyrin catalyst to co-catalyst is from 1 : 1 to 1 : 100.

7. A process according to claim 6, characterised in that the mole ratio of manganese porphyrin catalyst to co-catalyst is from 1 : 5 to 1 : 50.

8. A process according to any preceding claim, characterised in that the process employs a mixture of a hydrophobic solvent and a hydrophilic solvent.

9. A process according to claim 8, characterised in that the hydrophobic solvent is dichloromethane and the hydrophilic solvent is acetonitrile.

10. A process according to claims 8 or 9, characterised in that the weight ratio of hydrophobic solvent to hydrophilic solvent is in the range of from 10 : 1 to 1 : 10.

11. A process according to any preceding claim, characterised in that the aromatic compound comprising a heterocyclic nitrogen atom is unsubstituted or substituted by one or more hydroxy, alkoxy, alkyl, and amino groups.

12. A process according to any preceding claim, characterised in that the mole ratio of hydrogen peroxide to oxidisable heterocyclic nitrogen atom is in the range of from 1 : 1 to 10 : 1.

13. A process according to claim 12, characterised in that the mole ratio of hydrogen peroxide to oxidisable heterocyclic nitrogen atom is in the range of from 2 : 1 to 6 : 1.

14. A process according to any preceding claim, characterised in that the mole ratio of aromatic compound comprising a heterocyclic nitrogen atom : manganese porphyrin catalyst is selected to be in the range of from 10 : 1 to 1000 : 1.

15. A process according to claim 14, characterised in that the mole ratio of aromatic compound comprising a heterocyclic nitrogen atom : manganese porphyrin catalyst is selected to be in the range of from 100 : 1 to 600 : 1.

16. A process according to any preceding claim, characterised in that the process is carried out at a temperature of from ambient to 75°C.

17. A process according to any preceding claim, characterised in that the hydrogen peroxide is employed as an aqueous solution diluted with a hydrophilic organic solvent.

18. A process according to claim 17, characterised in that the hydrophilic organic solvent is acetonitrile.

19. A process for the production of heterocyclic N-oxides by reaction between an aromatic compound comprising a heterocyclic nitrogen atom and hydrogen peroxide in the presence of a catalyst, a co-catalyst, and an organic solvent, characterised in that the catalyst comprises a manganese porphyrin selected from the group consisting of manganese tetrakis (2,6-dichlorophenyl) porphyrin chloride, manganese tetrakis (2,6-dichlorophenyl-4-sulphonic acid) porphyrin chloride and manganese tetrakis (4-methylpyridinium) porphyrin chloride, the co-catalyst is ammonium acetate and that the solvent is a mixture of dichloromethane and acetonitrile.
